# EUROPEAN PATENT SPECIFICATION

(11) **EP 1 581 077 B1**
(45) Date of publication and mention of the grant of the patent: **22.10.2008**
(21) Application number: 03732316.9
(22) Date of filing: 01.05.2003
(51) Int. Cl.: A45D 44/00

(54) **ARTICLE FOR SELECTION OF INDIVIDUALIZED PERSONAL CARE PRODUCTS**
VORRICHTUNG ZUM AUSWÄHLEN VON INDIVIDUALISIERTEN KÖRPERPFLEGEMITTEL
ARTICLE DE SELECTION DE PRODUITS DE SOINS PERSONNELS INDIVIDUALISES

(30) Priority: 07.01.2003 US 337466
(43) Date of publication of application: 05.10.2005
(73) Proprietor: Unilever PLC, London Greater London EC4P 4BQ (GB); Unilever N.V., 3013 AL Rotterdam (NL)
(72) Inventor: ORTIZ-VALERO, Carlos, Greenwich, CT 06830 (US); DEPIANO, John Jr, Burlington, MA 01803 (US); DZIERSK, Mark, Dieter, Winnetka, IL 60093 (US); EDDOWES, Miles, Hugh, Unilever R & D Edgewater, Edgewater, NJ 07020 (US); GIBFORD, Gail Eileen, Unilever H & P Care USA, Greenwich, CT 06830 (US); KENNEDY, Christine Elizabeth, Unilever H & P Care USA, Trumbull, CT 06611 (US); PANNOZZO, Anthony, Brookline, MA 02445 (US)
(74) Representative: Acham, Nicholas Clive
(86) International application number: PCT/EP2003/004707
(87) International publication number: WO 2004/060105

(56) References cited:
- EP-A- 1 202 209
- EP-A- 1 240 845
- US-A- 3 851 655
- US-A- 3 860 015
- US-A- 4 434 467

## Description

The invention concerns an article whereby consumers can self-evaluate the most suitable personal care products for their body. Such an article is described in EP 1 240 845.

"Mirror mirror on the wall, who's the fairest of them all?". So inquired the wicked witch of the Snow White story. Were the witch to have had benefit of a personalized skin analysis, perhaps the recommended cosmetic treatment could have elevated her to be the fairest. Alas, she blindly selected her cosmetics without an understanding of those most suitable to her skin type.

Clinique^{®} has for some years provided a slide rule type diagnosis system for in-store advice. Based on answers to certain questions, a customer is directed to the appropriate cosmetic product(s).

In-store product recommendation charts based on customer input have not been very successful. Some of the systems are complicated to operate and interpret. Other systems fail to attract attention. Better systems are necessary than heretofore known for providing an easy to operate, easy to understand and attention grabbing mechanism which can recommend personalized product(s).

In a first aspect of the invention, a diagnostic article is provided which includes:
(i) a two-way mirror;
(ii) a plurality of attribute selection sites arranged along or associated with the mirror, the sites each having a plurality of choice selectors;
(iii) an output for recommending at least one personal care product based upon input from the choice selectors, and wherein the at least one personal care product is identified by an icon, pictorial or trademark on a support behind the two-way mirror; and
(iv) a backlight behind the mirror arranged to selectively highlight the icon, pictorial or trademark.

Attribute selection sites for skin products may be those hosting questions related to a customer's age, skin coloration, dryness, sunburn susceptibility, pimple breakout affinity, wrinkle formation, pore size and combinations thereof. Advantageously, there can be from two to twenty but preferably from three to six choice selectors within each of a plurality of attribute selection sites. The sites may range in number from three to thirty, preferably from four to eight.

Activation of one of the selectors in a site will in preferred embodiments preclude concurrent activation of any other selector at that same site. Each of the choice selectors within a site may differ from another by representing a different degree of the attribute describing the site.

Selectors may be activated by finger pressure against an area designated by a writing as a selector site. Activation of a selector can initiate an electronic transmission signal to an electronic computer. Alternatively activation of a selector can initiate a mechanical transmission not connected to an electrical output. A low cost form of the output according to the present invention involves use of charts. This may be constructed of either paperboard or plastic. The chart may be a wheel identifying a plurality of personal care products at different locations on the wheel. Alternatively it may be a flip-chart of multiple pages or screens held together by a binder such as a loose-leaf ring.

A most important part of the present invention is that of a mirror which is a two-way mirror. Each of a plurality of personal care products is identified by an icon (e.g. a pictorial or word mark) which is supported on a screen behind the mirror. A backlight behind the mirror is so arranged to selectively highlight one or more icons. The area of highlighting will relate to the product recommended through a questionnaire.

A still further alternative embodiment is where a membrane keypad is held behind the mirror. Entry of answers to the questionnaire are then indicated by pressing appropriate keys on the membrane keypad.

Further features and advantages of the present invention will become more apparent by consideration of the following drawings in which:
Fig. 1 is a mechanically operated first embodiment of the diagnostic article according to the present invention;
Fig. 2 is a product finder reference chart utilized in combination with the first embodiment;
Fig. 3 is a perspective view of the first embodiment shown arranged on a store shelf;
Fig. 4 is an electronically operated second embodiment of a diagnostic article according to the present invention;
Fig. 5 is a schematic cross-sectional view of the internal mechanism taken along line 5-5 of the second embodiment shown in Fig. 4;
Fig. 6 is an electronically operated third embodiment of a diagnostic article according to the present invention; and
Fig. 7 is the third embodiment according to Fig. 6 which has been activated by customer input to reveal those personal care products most suitable for this particular customer.

Now there has been found a diagnostic article which overcomes many of the disadvantages found with previously known ones. No longer is the diagnostic article lacking features of natural human attraction or forbiddingly uninviting. A mirror forms the central focus of the present invention. Humans are invariably attracted to a mirror. For some reason a person's own image is irresistible to view. Once the customer has been attracted to the display, the diagnostic array of questions comes to the customer's attention and is less daunting than a stand alone questionnaire/recommendation device.

Additionally, the mirror assists a customer in answering certain questions concerning their personal attributes. For instance, a question requesting identification of a person's skin tone becomes easier to answer. A series of different tones may be depicted on or directly adjacent to the mirror. The closest tone is much more easily identified through correlating the color of the face appearing in the mirror with one of the tones of the color array. Other attributes may also interact with feedback from a mirror image. Whether skin is oily or dry can be answered by viewing the mirror reflection.

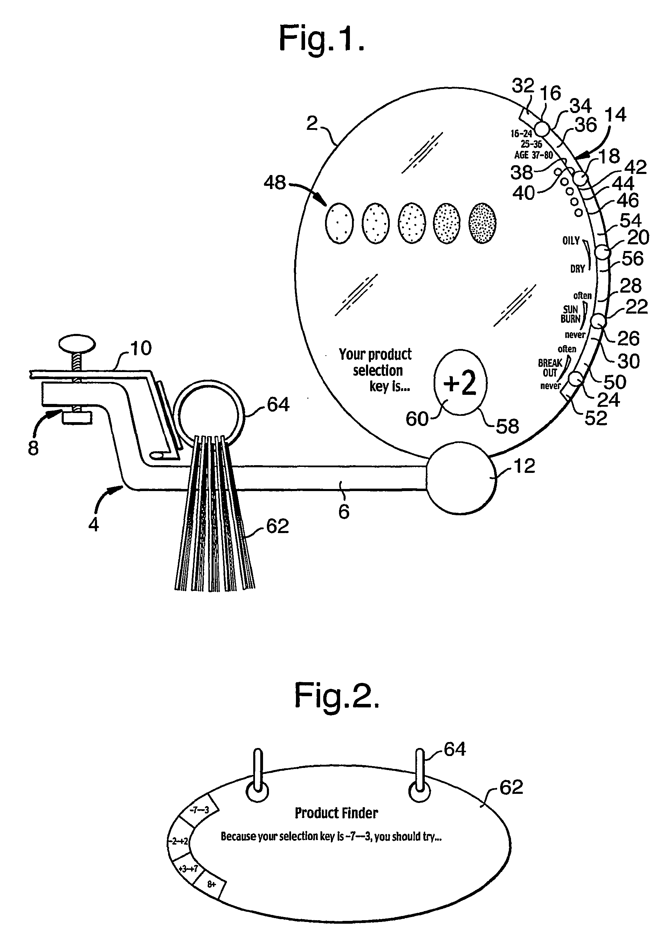

Fig. 1 illustrates a first embodiment of the present invention. A diagnostic article includes a mirror 2 attached to a support member 4. Elements of the support member include an arm 6 which at one end has an attachment member 8 in the form of a screwable bracket intended to be anchored onto a store shelf 10. Alternative support member embodiments would include a free-standing support base resting upon the shelf, a spring loaded clamp and an adhesive bond.

A connector member 12 is positioned at an opposite end of the arm distant from the attachment member and jutting outward beyond the shelf.

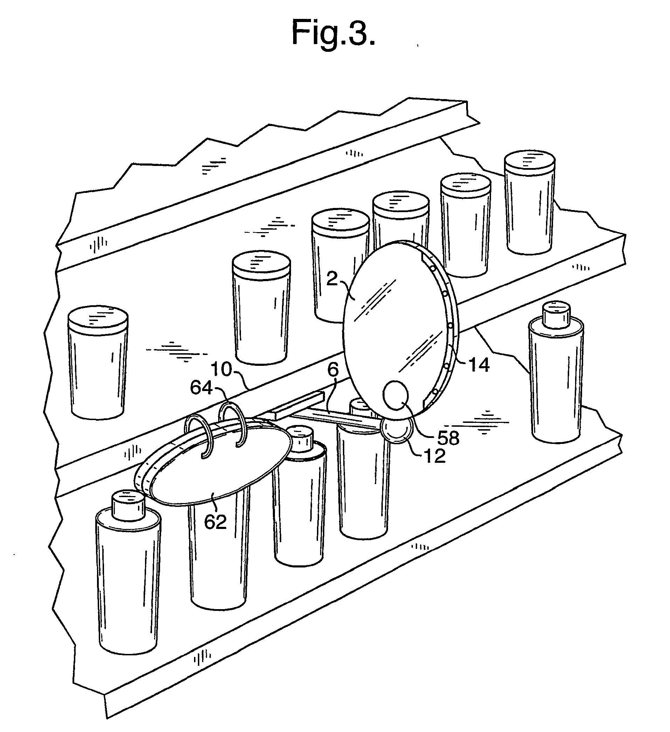

Fig. 3 best illustrates the diagnostic article in the context of a store shelf adjacent a variety of cosmetic containers.

The connector member may be movable or stationary and functioning to join the mirror to the support member. Moveability can be achieved by a ball-in-socket, spring clamp, ratchet, hinge or similar mechanism within the connector member.

The embodiment shown in Fig. 1 depicts five attribute selection sites along a selector track 14. These attribute selection sites include ones for age 16, color or tone 18, oiliness 20, sunburn susceptibility 22, and pimple breakout frequency 24.

A moveable lever 26 abuts each of the attribute selection sites. This lever can manually be manipulated to be placed adjacent one of the several choice selector positions. For instance, with respect to the sunburn susceptibility attribute selection site 22, there can be a choice between the "often" 28 and "never" 30 choice selector positions.

Another of the attribute selection sites relates to age. There are choice selector positions along this site for the age groups "16-24", "25-36" and "37-80" seen in Figure 1 as elements 32, 34 and 36.

Directly below is an attribute selection site for skin color or tone. A series of five shades of color or tone are presented at different choice selector positions 38, 40, 42, 44 and 46. An identical color or tone set 48 is arrayed in much larger form across a central face of the mirror. This enlarged set of color or tone on a prominent face of the mirror helps a customer match their skin as reflected in the mirror to one of the choice selector colors or tones. Guesswork is eliminated.

A fourth of the attribute selection sites is that for pimple breakout frequency. Along this question printed adjacent the mirror are two choice selector positions. These are for the answer "often" 50 or "never" 52. In some embodiments, there may be additional intermediate choice selector positions or a continuum of such positions.

The fifth of the attribute selection sites is that of oiliness 20. Here there may be choice selector positions of "oily" 54 and "dry" 56. There also may be several intermediate positions or a continuum of positions.

Upon a customer having arranged each of the movable levers to a choice selector position along the track, a mechanically operated pre-printed program board becomes oriented into a product selection window 58. In the embodiment of Fig. 1, the window is found at a lower section of the mirror. A key 60 appears within a window. The drawing illustrates the key as the number +2 which has been printed on the program board.

In its most particular form, the first embodiment involves an approximately 20.32cm (8 inch) mirror. Each movable lever 26 is interconnected along an 1 x 2.5 linkages which ultimately drive a wheel with the program board.

The next step for a customer is to consult a product finder set of cards 62 held within a ring binder 64. Fig. 2 illustrates one embodiment of a typical card attached to the ring binder. Either in word reference or through a pictorial icon, the product finder card correlates the key identified from the mirror into particular personal care product(s) personalized to the customer. The ring binder with cards can be hung from the same shelf and even supported on the same attachment member as the mirror.

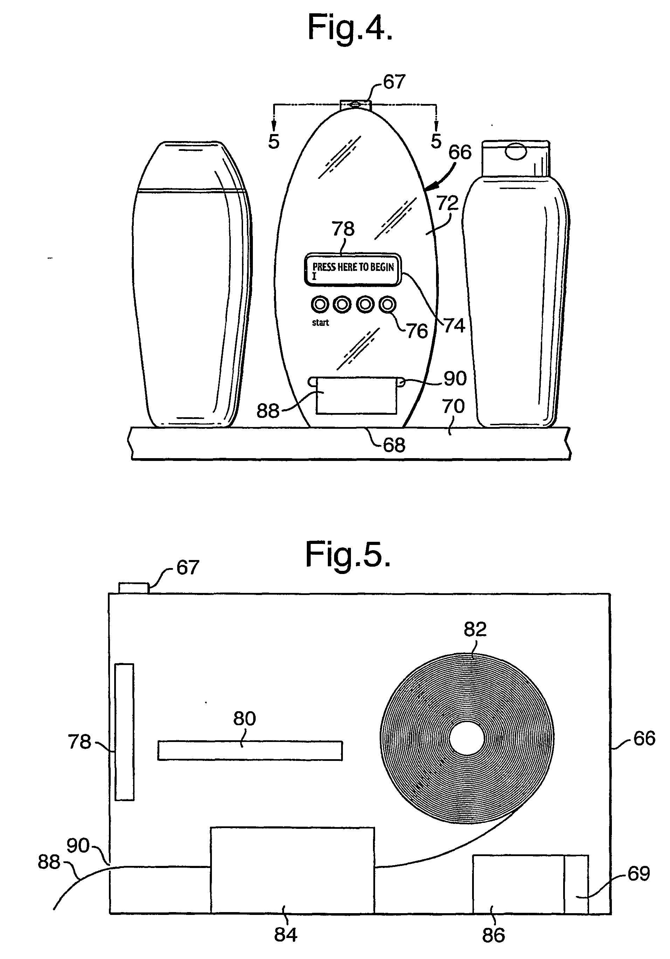

A second embodiment of the present invention is best illustrated in Fig. 4. A housing 66 consolidates the diagnostic article within a single easily portable device. The housing has a flat bottom wall 68 capable of free-standing on a shelf 70. A front face of the housing is a mirror 72.

A window 74 and a series of choice selectors in the form of buttons 76 are formed on the front mirror face of the housing. A LCD screen 78 is displayed within window 74. Supported inside the housing is a program on a computer board 80.

A series of questions are stored in the program. These questions relate to personal questions such as age, skin coloration, dryness, sunburn susceptibility, pimple breakout affinity, wrinkles and pore sizes. When these appear within the window on the LCD screen, each question is considered an attribute selection site within the context of this invention. The customer is requested to answer each attribute question by pressing one of the four buttons 76. For instance, the screen may request the customer's age and list four choices such as "16+", "24+", "35+" and "50+", each positioned over one of the four buttons. Color or skin tone may be depicted on the LCD screen as a color icon or in words such as mocha, olive, light honey and ivory. The mirror helps the customer decide by viewing their own face and thereupon providing input to the questions.

Once a customer has answered all attribute questions, the program then calculates which is the best personal care product or set of products personalized to the customer.

Alternatively or in addition to the LCD screen printout of best product set, a paper printout 88 can be provided describing the best product(s). Fig. 5 illustrates the printing mechanism. A paper roll 82 is supported within housing 66. Paper is fed from the roll to a printer 84. Power is generated by a battery unit 86. Printout 88 exits the housing through a slot 90. The customer can then tear off the printout from the paper roll and retain as a reminder or personal record.

Advantageously the diagnostic article according to the present invention may further include a camera 67 aligned with the mirror 72 for taking an image of the same customer face as mirror reflected. A still further optional aspect of the present invention is that of a wireless phone 69. This phone should be capable of transmitting to a distant central computer such as a central headquarters information provided by the customer. The phone connection will allow retransmission from the central location back to the diagnostic article and provide greater informational capability than would be available merely from the store diagnostic equipment.

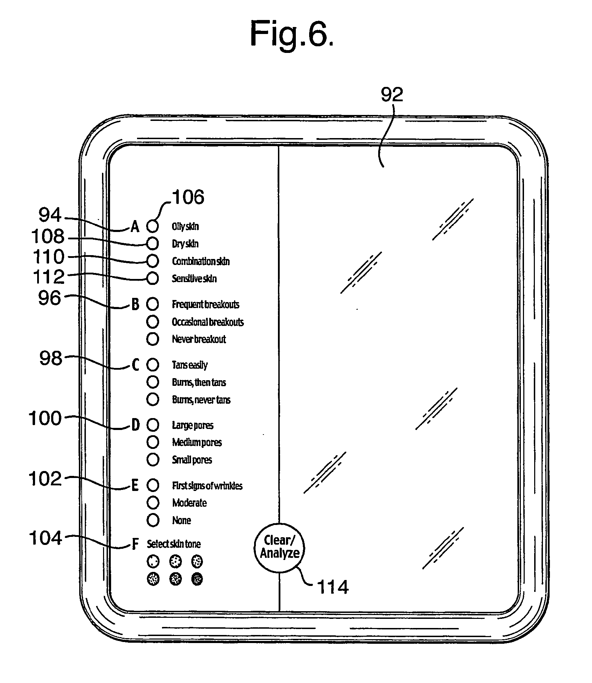

Fig. 6 illustrates a third embodiment of the present invention. As with all other embodiments, a mirror 92 is the focus of attention. A series of six attribute selection sites 94-104 are arrayed in a column down a border of the mirror. These sites correspond to questions regarding dryness, pimple breakout affinity, sunburn susceptibility, pore size, wrinkles and skin coloration (and tone), respectively. The question posed for each attribute selection site has from three to six possible answers in the form of choice selector buttons. For instance, the skin dryness attribute site 94 has four possible choice selectors 106-112. These answers may range from oily skin, dry skin, combination skin and sensitive skin, respectively.

The skin coloration or tone attribute site 104 depicts the selection as six colored circles corresponding to a skin tone. Each of these choice selector colors represent an activatable button.

Once all of the questions have been answered by a customer applying finger pressure on an appropriate choice selector, the analyze button 114 is pressed. A pre-programmed computer then provides a recommendation to several personalized products that can be used in a skin care regime. This regime is tailored to the type of skin which the customer has described in their answer response.

Mirror 92 is a two-way mirror which underneath the buttons has a digital keypad for transmitting information to a miniaturized computer network. Anywhere from six to twenty-four regimes can be recommended. Within each regime are anywhere from one to six different types of products such as a toner, cleanser, moisturizer and/or conditioner. Color cosmetics may also be recommended and can include facial foundation, lipstick, mascara, blush, eyeliner and nail polish.

Icons depicting the recommended products are placed on transparencies 115 beneath the two-way mirror. The personalized set of products is presented as icons selectively backlighted so that their images shine through the two-way mirror.

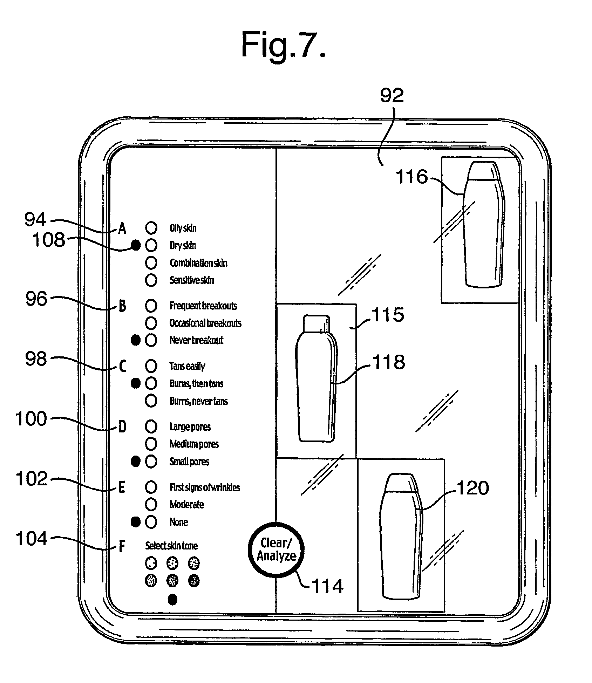

Fig. 7 illustrates a backlighting sequence. One of each of the chosen choice selectors of the attribute selection sites is backlighted as well as the analyze button 114. This refreshes the customer's memory and confirms the selected answers to the questions along the attribute selection sites. Secondly, backlighting highlights three products suitable for the person pursuant to the questionnaire analysis. Backlighted depictions are presented of bottles for a facial cleanser 116, a body shower gel 118 and a conditioning lotion 120.

Variations of the third embodiment can also be practiced according to the present invention. For instance, the two-way mirror embodiment of Fig. 6-7 can be provided with a membrane keypad underneath the mirror. Questions and a list of potential responses are viewable by scrolling up and down the mirror. The selector choices can be highlighted by an LCD light. Based upon the individual choice selector response, a set of personal care products are then recommended. The recommendation is accomplished by triggering a color LCD image behind the two-way mirror. The image of a product bottle can then shine through the mirror. Those icons representing other (non-appropriate) products remain dark and therefore, non-visible to the customer.

Diagnostic articles according to the present invention are particularly suitable for skin but are not limited thereto. For instance, the device of this invention may be applicable to hair, underarm and oral products. Among questions necessary to probe for an appropriate personalized regime for hair treatment include those related to oiliness, color, dandruff susceptibility, age, curliness and thickness. Hair products can be recommended from categories including personalized conditioner, shampoo, styling aid, colorants and hair sprays.

Dental products can also be personalized with the diagnostic article of this invention. Questions that may be placed to a customer include teeth whiteness, number of cavities, breath freshness, taste, packaging type and combinations thereof. Use of the mirror is particularly effective to help customers match their teeth color in response to a question(s) regarding coloration. The mirrors also are useful to have a customer identify the number of cavities which may have been previously filled.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

## Claims

1. A diagnostic article comprising:
(i) a two-way mirror (2);
(ii) a plurality of attribute selection sites (16, 18, 20, 22, 24) arranged along or associated with the mirror (2), the sites each having a plurality of choice selectors;
(iii) an output (62) for recommending at least one personal care product based upon input from the choice selectors, and wherein the at least one personal care product is identified by:an icon, pictorial or trademark on a support behind the two-way mirror; and
(iv) a backlight behind the mirror (2) arranged to selectively highlight the icon, pictorial or trademark.

2. The article according to claim 1 wherein the attribute selection sites are areas hosting questions related to those selected from age, skin coloration, dryness, sunburn susceptibility, pimple breakout affinity, wrinkles, pore sizes and combinations thereof.

3. The article according to claim 1 or claim 2 wherein a plurality of the sites have at least three choice selectors.

4. The article according to any of the preceding claims wherein activation of one of the choice selectors in a site precludes concurrent activation of any other selector at that same site.

5. The article according to any of the preceding claims wherein each of the choice selectors within a site differ from one another by representing a different degree of an attribute describing the site.

6. The article according to any of the preceding claims wherein a choice selector is activated by finger pressure against an area designated as a selector site.

7. The article according to any of the preceding claims wherein activation of a choice selector initiates an electronic transmission signal to a computer program.

8. The article according to any of the preceding claims wherein activation of a choice selector initiates a mechanical transmission not connected to an electronic computer.

9. The article according to any of the preceding claims wherein the output is detailed on one or more charts which recommend one or more personal care products most suitable to a customer.

10. The article according to any of the preceding claims further comprising a membrane keypad behind the mirror.

11. The article according to any of the preceding claims wherein the attribute selection sites are areas hosting hair related questions selected from the group consisting of oiliness, color, dandruff susceptibility, age, curliness, thickness and combinations thereof.

12. The article according to any of the preceding claims wherein the attribute selection sites are areas hosting dental questions related to those selected from the group consisting of teeth whiteness, number of cavities, breath freshness, taste, packaging and combinations thereof.

13. The article according to any of the preceding claims further comprising a camera aligned with the mirror focusing on a customer whose image is reflected in the mirror.

14. The article according to any of the preceding claims further comprising a wireless phone transmitting information processed from input from the choice selectors and the information being transmitted to a distant computer.

15. The article according to any of the preceding claims further comprising an array of similarly sized areas each with a different color shade arranged on the mirror for helping to find a color match with that of a facial image reflected in the mirror.

## Patentansprüche

1. Diagnoseartikel, der umfasst:
(i) einen Zweiwegespiegel (2);
(ii) mehrere Attributauswahlstellen (16, 18, 20, 22, 24), die längs des Spiegels (2) oder in Zuordnung zu ihm angeordnet sind, wobei die Stellen jeweils mehrere Auswahleinrichtungen besitzen;
(iii) eine Ausgabe (62), um wenigstens ein persönliches Pflegeprodukt anhand der Eingabe von den Auswahleinrichtungen zu empfehlen, wobei das wenigstens eine persönliche Pflegeprodukt durch ein Icon, eine bildliche Darstellung oder eine Handelsmarke auf einem Träger hinter dem Zweiwegespiegel identifiziert wird; und
(iv) eine Hintergrundbeleuchtung hinter dem Spiegel (2), die dazu ausgelegt ist, das Icon, die bildliche Darstellung oder die Handelsmarke wahlweise hervorzuheben.

2. Artikel nach Anspruch 1, wobei die Attributauswahlstellen Bereiche sind, die Fragen enthalten, die auf jene bezogen sind, die aus dem Alter, der Hautfarbe, der Trockenheit, der Sonnenbrandempfindlichkeit, der Pickelausbruchsaffinität, den Falten, den Porengrößen und Kombinationen hiervon ausgewählt sind.

3. Artikel nach Anspruch 1 oder Anspruch 2, wobei mehrere Stellen wenigstens drei Auswahleinrichtungen besitzen.

4. Artikel nach einem der vorhergehenden Ansprüche, wobei die Aktivierung einer der Auswahleinrichtungen an einer Stelle eine gleichzeitige Aktivierung einer anderen Auswahleinrichtung an derselben Stelle ausschließt.

5. Artikel nach einem der vorhergehenden Ansprüche, wobei sich alle Auswahleinrichtungen an einer Stelle durch die Darstellung eines anderen Grades eines die Stelle beschreibenden Attributs voneinander unterscheiden.

6. Artikel nach einem der vorhergehenden Ansprüche, wobei eine Auswahleinrichtung durch Fingerdruck gegen einen Bereich, der als eine Auswahlstelle bezeichnet ist, aktiviert wird.

7. Artikel nach einem der vorhergehenden Ansprüche, wobei eine Aktivierung einer Auswahleinrichtung ein elektronisches Übertragungssignal zu einem Computerprogramm auslöst.

8. Artikel nach einem der vorhergehenden Ansprüche, wobei eine Aktivierung einer Auswahleinrichtung eine mechanische Übertragung, die nicht mit einem elektronischen Computer verbunden ist, auslöst.

9. Artikel nach einem der vorhergehenden Ansprüche, wobei die Ausgabe in eine oder in mehrere Tabellen unterteilt ist, die ein oder mehrere persönliche Pflegeprodukte, die für einen Kunden am besten geeignet sind, empfehlen.

10. Artikel nach einem der vorhergehenden Ansprüche, der ferner ein Membrantastenfeld hinter dem Spiegel umfasst.

11. Artikel nach einem der vorhergehenden Ansprüche, wobei die Attributauswahlstellen Bereiche sind, die auf die Haare bezogene Fragen enthalten, die aus der Gruppe ausgewählt sind, die besteht aus Fettigkeit, Farbe, Schuppenanfälligkeit, Alter, Lockenbildung, Dicke und Kombinationen hiervon.

12. Artikel nach einem der vorhergehenden Ansprüche, wobei die Attributauswahlstellen Bereiche sind, die auf Zähne bezogene Fragen enthalten, die mit jenen in Beziehung stehen, die aus der Gruppe ausgewählt sind, die besteht aus Zahnweißheit, Anzahl von Lücken, Atemfrische, Geschmack, Anordnungsdichte und Kombinationen hiervon.

13. Artikel nach einem der vorhergehenden Ansprüche, der ferner eine Kamera enthält, die auf den Spiegel ausgerichtet ist und auf einen Kunden fokussiert ist, dessen Bild in dem Spiegel reflektiert wird.

14. Artikel nach einem der vorhergehenden Ansprüche, der ferner ein drahtloses Telephon enthält, das Informationen überträgt, die, wenn sie von den Auswahleinrichtungen eingegeben werden, verarbeitet werden, wobei die Informationen zu einem entfernten Computer übertragen werden.

15. Artikel nach einem der vorhergehenden Ansprüche, der ferner eine Anordnung von Bereichen mit ähnlicher Größe umfasst, wovon jeder eine andere Farbschattierung hat und auf dem Spiegel angeordnet ist, um das Auffinden einer Farbübereinstimmung mit der Farbe eines in dem Spiegel reflektierten Bildes eines Gesichts zu unterstützen.

## Revendications

1. Article de diagnostic, comprenant :
(i) un miroir semi-réfléchissant (2) ;
(ii) une pluralité de sites de sélection d'attributs (16, 18, 20, 22, 24) qui sont disposés le long du, ou qui sont associés au, miroir (2) ; les sites comprenant chacun une pluralité de sélecteurs de choix ;
(iii) une sortie (62) pour recommander au moins un produit de beauté personnalisé sur la base de l'entrée résultant des sélecteurs de choix, et dans lequel le au moins un produit de soin personnalisé est identifié par une icône, un dessin ou une marque commerciale appliqué(e) sur un support placé à l'arrière du miroir semi-réfléchissant ; et
(iv) un rétro-éclairage derrière le miroir (2) qui est placé de façon à mettre en surbrillance l'icône, le dessin ou la marque commerciale de manière sélective.

2. Article selon la revendication 1, dans lequel les sites de sélection d'attributs sont des zones qui hébergent des questions associées à celles qui se rapportent à l'âge, à la coloration de la peau, à la sécheresse de la peau, à la sensibilité aux coups de soleil, à la sensibilité aux éruptions cutanées, aux rides, à la taille des pores, ainsi qu'à une combinaison de toutes ces caractéristiques.

3. Article selon la revendication 1 ou la revendication 2, dans lequel une pluralité des sites possède au moins trois sélecteurs de choix.

4. Article selon l'une quelconque des revendications précédentes, dans lequel une activa-tion de l'un des sélecteurs de choix dans un site empêche une activation concomitante d'un autre sélecteur au niveau du même site.

5. Article selon l'une quelconque des revendications précédentes, dans lequel chacun des sélecteurs de choix à l'intérieur d'un site diffère des autres en ce qu'il représente un degré différent d'un attribut décrivant le site.

6. Article selon l'une quelconque des revendications précédentes, dans lequel un sélecteur de choix est activé en exerçant une pression du doigt contre une zone désignée comme un site de sélection.

7. Article selon l'une quelconque des revendications précédentes, dans lequel une activation d'un sélecteur de choix déclenche l'envoi d'un signal de transmission électronique à un programme d'ordinateur.

8. Article selon l'une quelconque des revendications précédentes, dans lequel une activation d'un sélecteur de choix déclenche une transmission mécanique qui n'est pas reliée à un ordinateur électronique.

9. Article selon l'une quelconque des revendications précédentes, dans lequel la sortie est détaillée sur un ou plusieurs tableaux qui recommandent un ou plusieurs produits de beauté personnalisés sensés être les mieux adaptés à un consommateur.

10. Article selon l'une quelconque des revendications précédentes, comprenant par ailleurs un bloc de touches à membrane derrière le miroir.

11. Article selon l'une quelconque des revendications précédentes, dans lequel les sites de sélection d'attributs sont des zones qui hébergent des questions associées à la chevelure, qui sont sélectionnées dans le groupe se rapportant à la facilité avec laquelle les cheveux graissent, à des cheveux colorés, qui développent des pellicules ; et se rapportant également à l'âge, au fait que les cheveux sont ondulés - ou non -, à leur grosseur ; ainsi qu'à une combinaison de ces caractéristiques.

12. Article selon l'une quelconque des revendications précédentes, dans lequel les sites de sélection d'attributs sont des zones qui hébergent des questions associées à la dentition, qui sont sélectionnées dans le groupe se rapportant à la blancheur des dents, au nombre de cavités, à la fraîcheur de l'haleine ; ainsi qu'au goût et à l'emballage des produits ; et à une combinaison de ces caractéristiques.

13. Article selon l'une quelconque des revendications précédentes, comprenant par ailleurs une caméra qui est alignée avec le miroir et qui est centrée sur un client dont l'image se reflète dans le miroir.

14. Article selon l'une quelconque des revendications précédentes, comprenant par ailleurs un téléphone sans fil qui transmet des informations traitées à partir de l'entrée délivré en sortie par les sélecteurs de choix ; les informations étant transmises à un ordinateur distant.

15. Article selon l'une quelconque des revendications précédentes, comprenant par ailleurs un ensemble de zones ayant les mêmes dimensions, qui sont dotées chacune d'une teinte de couleur différente disposée sur le miroir afin de faciliter une mise en correspondance de la couleur avec celle d'une image du visage qui se reflète dans le miroir.
